# FASCICULE DE BREVET EUROPEEN

(11) **EP 1 601 587 B1**
(45) Date de publication et mention de la délivrance du brevet: **14.06.2006**
(21) Numéro de dépôt: 04716627.7
(22) Date de dépôt: 03.03.2004
(51) Int. Cl.: B65D 83/02

(54) **DISPOSITIF DE DISTRIBUTION D'OBJETS DE FORME OBLONGUE AYANT UNE OUVERTURE PRINCIPALE ET AU MOINS UNE AUTRE OUVERTURE DE FORME ALLONGEE**
VORRICHTUNG ZUM VERTEILEN VON LÄNGLICHEN GEGENSTÄNDEN MIT EINER HAUPTÖFFNUNG UND MINDESTENS EINER WEITEREN LÄNGLICHEN ÖFFNUNG
DEVICE FOR DISPENSING OBLONG OBJECTS, COMPRISING ONE MAIN OPENING AND AT LEAST ONE OTHER ELONGATED OPENING

(30) Priorité: 03.03.2003 FR 0302729; 03.03.2003 FR 0302728
(43) Date de publication de la demande: 07.12.2005
(73) Titulaire: Airsec S.A.S., 94600 Choisy Le Roy (FR)
(72) Inventeur: LANCESSEUR, Didier, F-92100 Boulogne (FR); NOBILET, Roger, F-77500 Chelles (FR)
(74) Mandataire: Gaucherand, Michel
(86) Numéro de dépôt international: PCT/FR2004/000487
(87) Numéro de publication internationale: WO 2004/080366

(56) Documents cités:
- DE-U- 8 437 898
- FR-A- 2 624 106
- US-A- 5 788 064
- US-B1- 6 497 845
- PATENT ABSTRACTS OF JAPAN vol. 1998, no. 04, 31 mars 1998 (1998-03-31) -& JP 09 315455 A (NIFCO INC), 9 décembre 1997 (1997-12-09)
- PATENT ABSTRACTS OF JAPAN vol. 2003, no. 08, 6 août 2003 (2003-08-06) -& JP 2003 118758 A (MATSUSHITA ELECTRIC IND CO LTD), 23 avril 2003 (2003-04-23)

## Description

### Domaine de l'invention

L'invention concerne un dispositif de distribution d'objets de forme oblongue à section en particulier sensiblement polygonale, circulaire, elliptique et le conteneur équipé d'un tel dispositif de distribution.

De nombreux objets ayant une forme à section en particulier sensiblement polygonale, circulaire, elliptique, allongée sont utilisés comme consommables. De tels objets sont notamment des languettes, des « strip » tests en forme de bandelettes ou lamelles rigides utilisées par exemple à des fins de diagnostic ou de contrôle dans le domaine médical. D'autres objets à distribuer ayant une forme oblongue peuvent également être par exemple des pansements ou des produits alimentaires tels que des chewing-gums, des cure-dents, bâtonnets ou autres.

Pour des raisons évidentes, en particulier d'hygiène, mais aussi afin d'éviter toute dégradation et ainsi améliorer la durée de conservation des objets, ceux-ci sont placés à l'abri des pollutions extérieures et/ou des attaques physico-chimiques résultant du niveau d'humidité relative, de la lumière, en particulier des rayons UV et autres substances chimiques, ou encore des dégradations par effet mécanique.

Pour des raisons également d'hygiène, de conservation et de protection de nombreux dispositifs de distribution ont été imaginés afin de permettre une distribution unité par unité des objets de forme oblongue, de manière à distribuer le nombre exact d'objets requis et éviter ainsi toute pollution due à une sortie involontaire d'objets.

De tels dispositifs de distribution d'objets doivent être simples, peu onéreux à produire et d'une utilisation aisée.

### Etat de la technique

De nombreux dispositifs de distribution d'objets de forme sensiblement plate sont décrits dans la littérature technique, en particulier dans celle constituée par les demandes de brevet et/ou brevets publiés.

Selon un premier document de l'art antérieur (US 5,505 308), est décrit un dispositif de stockage de lamelles de test sensibles à l'humidité.

Le dispositif de stockage apparaît être composé d'un conteneur comportant une matière déshydratante et d'un couvercle assemblé de manière à pouvoir être dégagé de l'ouverture dudit conteneur dans un mouvement de translation suivi d'un mouvement de rotation.

Lors de sa mise en oeuvre, le couvercle est dégagé de l'ouverture du conteneur par un mouvement de translation dans le sens parallèle au sens d'extraction des lamelles de test, puis est soumis à rotation afin de permettre l'accès aux lamelles de test. Lesdites lamelles de test sont elles-mêmes partiellement pulsées vers l'extérieur du conteneur par l'intermédiaire d'un ressort, de manière à en permettre la préhension par l'utilisateur.

Ce dispositif de distribution possède de nombreux désavantages techniques comme, par exemple, le fait que plusieurs lamelles sont simultanément poussées hors du conteneur, augmentant dès lors le risque de contamination et/ou de détériorations mécaniques des lamelles de test non extraites à chaque nouvelle ouverture du conteneur. De plus, au moment de la préhension d'une lamelle test, les doigts de l'utilisateur ou les outils de préhension des lamelles viennent au contact de plusieurs de ces lamelles, au risque de dégrader, ou de contaminer par pollution externe, les surfaces actives de ces lamelles.

Ce dispositif est également complexe et relativement coûteux à produire car il requiert l'usage d'un dispositif d'extraction des lamelles de test par l'intermédiaire d'un ressort et d'un couvercle à cinématique complexe. Un autre inconvénient de ce système est qu'il requiert généralement, lors de son ouverture, l'usage des deux mains, le rendant relativement peu maniable.

Dans un autre document (US5,788,064), est décrit un dispositif de distribution de lamelles test comportant un élément dessicant et un couvercle qui, une fois fermé, ne laisse pas passer l'humidité. A la différence d'autres dispositifs, en particulier celui précédemment évoqué, ce dispositif ne dispose pas d'un moyen de distribution des lamelles de test destiné à les pousser, la distribution se faisant par renversement du conteneur. Au moment où est pratiquée l'ouverture, le couvercle du conteneur bascule par rotation autour d'un axe perpendiculaire à l'axe de distribution des lamelles, de manière à dégager partiellement l'ouverture du conteneur et permettre la sortie des lamelles. Les lamelles sortant du conteneur sont déviées vers une butée au moyen d'une surface du couvercle disposée face à l'ouverture empêchant ainsi les lamelles de tomber.

De la même manière que pour le dispositif décrit dans le brevet US5,505,308, ce dispositif de distribution possède des désavantages liés au fait que, lors de l'ouverture du conteneur, plusieurs lamelles peuvent être extraites simultanément, sans pouvoir en contrôler le nombre, risquant ainsi de détériorer involontairement certaines lamelles de test réintégrées dans le conteneur. Un autre inconvénient de ce dispositif est lié au fait que l'ouverture permettant l'extraction des lamelles est de taille importante, augmentant ainsi le risque de contamination de l'intérieur du conteneur.

Un autre document (PATENT ABSTRACT OF JAPAN, vol 1998, n° 04) décrit un dispositif de distribution, unité par unité, de comprimés pharmaceutiques, comportant:
- une ouverture centrale circulaire définie par des pièces souples présentant chacune une forme en trapèze et disposées de manière à former une surface tronconique, ladite ouverture centrale circulaire présentant un diamètre égal à celui du composé à extraire,
- des languettes flexibles de forme rectangulaire, placées entre chaque pièce souple et de longueur supérieure aux coté de chaque pièce souple, lesdites languettes flexibles débouchant dans l'ouverture centrale circulaire et formant une deuxième ouverture circulaire, de diamètre inférieur à celui du composé à extraire et coaxiale avec la première.

L'extraction des comprimés unité par unité se fait, en retournant et en secouant le conteneur, lesdits comprimés étant guidés jusqu'à l'ouverture centrale circulaire par l'effet des surfaces courbées de pente constante formant la surface tronconique, par élargissement du passage défini par lés languettes flexibles. Après extraction d'un comprimé, les languettes flexibles retrouvent leur position et leur forme initiales.
Ainsi, ce dispositif de distribution est destiné à la distribution d'objet du type comprimé à enveloppe cylindrique et donc absolument inadapté à la distribution d'objet oblongues selon l'invention examinée.

Aucun des dispositifs de l'art antérieur ne donne de résultats satisfaisants car ces dispositifs ne permettent pas de contrôler le nombre d'objets ou lamelles extraits du conteneur. En outre, de tels dispositifs ne permettent pas de garantir l'absence de pollution ou de salissures des lamelles inopinément extraites puis réintroduites dans le conteneur car non utilisées en raison de leur surnombre, détériorant ainsi la qualité du stockage des lamelles. De plus, les dispositifs de distribution de l'art antérieur sont souvent composés de nombreuses pièces mécaniques qui augmentent à la fois leur coût de production et qui créent des difficultés de réalisation et de manipulation de ces dispositifs.

### Exposé de l'invention

Un problème posé est de réaliser un dispositif de distribution d'objets de forme oblongue à section en particulier sensiblement polygonale, circulaire, elliptique, tels que par exemple des lamelles ou des bandelettes rigides, qui permette non seulement la distribution de ces objets unité par unité, mais encore de résoudre tout ou partie des inconvénients précédemment évoqués.

Seul un objet sélectionné et distribué doit entrer en contact avec le milieu extérieur du conteneur, les autres objets restant à l'abri à l'intérieur du conteneur.

Un tel dispositif de distribution doit être adapté pour pouvoir être manoeuvré d'une seule main, facilitant ainsi sa manipulation, l'autre main étant libre pour assurer la préhension de l'objet distribué.

Dès lors, l'invention concerne d'abord un dispositif de distribution d'objets de forme oblongue à section en particulier polygonale, circulaire, elliptique, permettant la distribution unité par unité desdits objets à distribuer, tel que défini dans la revendication 1.

L'invention concerne également un conteneur contenant les objets oblongs à distribuer à l'une des extrémités duquel est monté le dispositif de distribution.

Ainsi, un tel dispositif associé à un conteneur s'utilise pour séparer et distribuer unité par unité un stock d'objets à distribuer se trouvant stockés en attente d'utilisation pour être protégés du milieu extérieur.

Lorsque l'opération de distribution est requise, l'utilisateur inverse le sens du dispositif de distribution par retournement, de telle manière que le stock d'objets à distribuer se trouve placé au-dessus dudit dispositif de distribution au contact de sa surface antérieure.

Par l'inversion de sens du dispositif et dès lors, par l'action de la gravité, les objets à distribuer viennent successivement en contact :
- d'abord, avec les surfaces de guidage disposées de part et d'autre des ouvertures allongées. Chaque surface de guidage a pour fonction de positionner les objets à distribuer de manière à ce que l'une des extrémités des objets oblongs à distribuer soit rendue sensiblement parallèle à l'ouverture de forme allongée située à proximité de la surface de guidage,
- puis avec la ou les ouverture(s) de forme allongée, pouvant avoir l'aspect d'une fente, pratiquée(s) au travers du dispositif de distribution.

Les objets à distribuer, animés d'un mouvement provoqué par l'inversion de sens et le choc de contact entre les objets à distribuer et les surfaces de guidage sur la face antérieure du dispositif de distribution, sont collectés et dirigés vers l'une au moins des ouvertures allongées, glissent le long de ces ouvertures allongées en direction de l'ouverture principale de distribution.

L'ouverture principale de distribution peut avoir une forme polygonale, circulaire ou ovale. Cette ouverture a des dimensions qui permettent le passage d'un seul objet à la fois. Ainsi dans le cas où l'ouverture principale de distribution est circulaire, son diamètre est généralement proche de la largeur ou du diamètre de l'objet à distribuer. Dès lors, quel que soit le nombre d'ouvertures allongées débouchant sur l'ouverture principale de distribution, un seul objet sera distribué à la fois.

Les ouvertures de forme allongée ont une longueur au moins égale à deux fois la largeur des objets à distribuer et préférentiellement au moins égale à trois fois la largeur desdits objets à distribuer.
Les ouvertures de forme allongée ont une largeur proche de l'épaisseur minimale de l'objet à distribuer, de telle manière que l'objet se positionne dans une ouverture allongée et soit préférablement légèrement tenu dans celle-ci, tout en glissant en direction de l'ouverture principale de distribution.

La fonction de collecte des objets à distribuer se fait dès lors, par l'intermédiaire de la ou des ouvertures allongées qui permettent de guider un ou plusieurs objets en direction de l'ouverture principale de distribution.

La fonction de sélection de l'objet à distribuer se fait dès lors principalement au niveau de l'ouverture principale de distribution. Lorsque plusieurs objets à distribuer sont guidés par la ou les ouverture(s) allongée(s) et parviennent en même temps dans la zone de l'ouverture principale de distribution, un seul objet peut être sélectionné et distribué au travers de cette ouverture principale de distribution, les autres objets restant bloqués à l'intérieur du conteneur.

Généralement, l'objet sélectionné s'engage et passe partiellement dans l'ouverture principale de distribution et y reste légèrement coincé, de telle sorte qu'il ne peut tomber hors du conteneur. Dès lors, une partie de l'objet ainsi sélectionné par ce dispositif est placée hors du conteneur, devient accessible à l'utilisateur du côté de la surface postérieure du dispositif, permettant une préhension aisée de la partie de l'objet accessible, et l'extraction complète de l'objet hors du conteneur.

La distribution d'un nouvel objet se fait en retournant ou en secouant à nouveau le dispositif de distribution de manière à orienter le dispositif vers le bas de telle sorte que le stock d'objets à distribuer soit au contact de la surface antérieure du dispositif.

L'ouverture principale de distribution est placée en un point quelconque de la surface du dispositif de distribution, et peut être placée tangentiellement au périmètre externe dudit dispositif. Toutefois cette ouverture est préférentiellement coaxiale par rapport à l'axe de symétrie du dispositif de distribution.

D'autres avantages du distributeur d'objets oblongs selon l'invention apparaîtront à la lecture de l'exemple de réalisation détaillé de l'invention, en se référant aux dessins donnés à titre d'illustration, dans lequel :
- la figure 1 représente une vue en perspective d'un conteneur de distribution équipé de son dispositif de distribution ;
- la figure 2 représente une vue en perspective d'un dispositif de distribution seul ;
- la figure 3 représente un dispositif de distribution en vue de dessus ;
- la figure 4 représente une vue en coupe selon l'axe A-A du dispositif de distribution ;
- la figure 5 représente une vue en coupe selon l'axe B-B du dispositif de distribution ;

### Description détaillée de l'invention

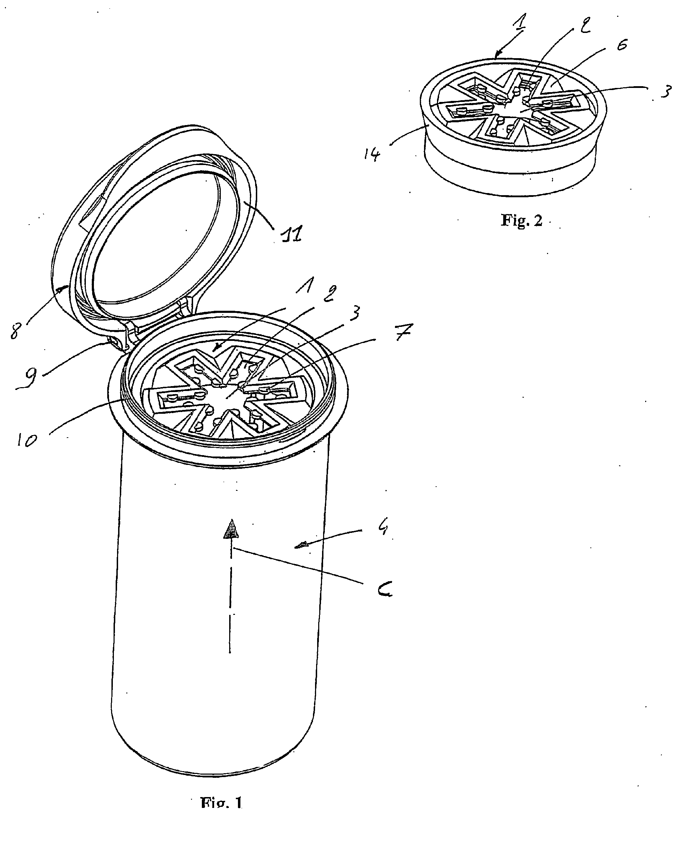

La figure 1 représente une vue en perspective du dispositif de distribution (1) ayant la forme d'un disque placé à l'intérieur du conteneur cylindrique de distribution (4) contenant les objets oblongs (13) à distribuer. Comme mentionné précédemment, les objets oblongs à distribuer sont, dans ce cas descriptif particulier, des lamelles de tests fréquemment utilisées dans le domaine des diagnostics médicaux ou analyses chimiques.

La figure 2 représente une vue en perspective détaillée du dispositif de distribution (1) en forme de disque, séparé de son conteneur.

Ce dispositif (1) est composé d'une ouverture principale de distribution (3) préférentiellement mais non exclusivement circulaire, sur laquelle débouche une série d'ouvertures allongées (2) disposées radialement autour de l'axe de l'ouverture principale de distribution (3).

Préférentiellement les ouvertures allongées sont régulièrement réparties autour de l'axe de symétrie de l'ouverture principale de distribution (3) et forment, par exemple, un X si quatre ouvertures allongées sont utilisées ou une étoile à six branches dans le cas où six ouvertures allongées seraient réparties autour de l'ouverture principale de distribution (3).

Le nombre d'ouvertures allongées (2) débouchant sur l'ouverture principale de distribution (3) n'est pas limité. Toutefois il a été constaté que la distribution des objets(13) est améliorée lorsque six ouvertures allongées sont régulièrement réparties autour de l'axe de symétrie principal d'une ouverture principale de distribution circulaire (3).

Généralement les ouvertures allongées (2), pratiquées au travers du dispositif de distribution, sont en forme de fentes dont les côtés sont sensiblement parallèles entre eux, conférant à l'ouverture allongée une largeur substantiellement constante. Les côtés longitudinaux des fentes sont espacés d'une distance sensiblement égale ou légèrement inférieure à l'épaisseur de l'objet oblong à distribuer de telle manière que celui-ci ne puisse passer totalement au travers de la fente qu'en y étant forcé mécaniquement, par une légère traction pratiquée sur la partie émergée du dispositif lors de l'acte de préhension.

Les ouvertures allongées (2) peuvent également être en forme de fentes de largeur légèrement croissante dans la direction allant de l'extrémité périmétrique de l'ouverture allongée vers l'ouverture principale de distribution (3). De préférence, la fente à largeur croissante est formée de deux côtés longitudinaux sensiblement rectilignes formant entre eux un angle légèrement ouvert en direction de l'ouverture principale de distribution, la valeur de cet angle étant généralement comprise dans l'intervalle allant de 0° à 10°, cette valeur étant ajustée en fonction du type d'objets distribués, des matériaux et des formes du dispositif de distribution.

En appliquant un mouvement sec au dispositif de distribution et de ce fait aux objets oblongs à distribuer placés à l'intérieur du conteneur, certains objets peuvent s'introduire, sous l'action de leur énergie cinétique, dans l'ouverture allongée en forme de fente à largeur croissante. Dans ce cas, et toujours sous l'action de cette même énergie cinétique, les objets viennent naturellement glisser en direction de l'ouverture principale de distribution car plus l'objet à distribuer se rapproche de l'ouverture principale de distribution et moins il est comprimé entre les côtés de la fente. Dans certains cas, les objets introduits dans une telle fente et qui n'ont pas été distribués restent coincés à l'endroit où l'espace entre les côtés de la fente est sensiblement égal à l'épaisseur de l'objet à distribuer. Cette fente à largeur croissante est donc avantageuse à la fois pour diriger les objets en direction de l'ouverture principale de distribution, mais aussi, dans certains cas, pour maintenir par léger coincement ou frottement, les objets introduits dans une des ouvertures allongées et non encore distribuées.

Les ouvertures allongées (2) peuvent avoir de multiples formes et être notamment rectangulaires. Des ouvertures allongées peuvent également avoir au moins un des cotés courbés, ou bien deux côtés courbés symétriquement, de telle manière que les cotés courbés forment une ouverture allongée en créant des rétreints par portions. Il est évident que de nombreuses formes d'ouvertures allongées peuvent être utilisées pour former un tel rétreint, sans départir de l'objet de l'invention. Le rétreint de l'ouverture allongée est généralement positionné au milieu de la longueur de l'ouverture, de manière à pincer légèrement et localement un des objets oblongs à distribuer.

Des zones des côtés des ouvertures (2), (3), en contact avec les objets à distribuer, peuvent comporter des sections ou des protubérances en matériaux élastiques afin d'améliorer la fonction de coincement des objets dans l'ouverture par le biais d'une compression élastique desdits objets. Les zones de contacts en matériaux élastiques des côtés des ouvertures allongées (2) peuvent être notamment des excroissances ponctuelles (7) comme celles représentées sur les figures 1 à 3. Les zones élastiques de contact des côtés des ouvertures peuvent également être positionnées sur tout ou partie de la longueur de l'ouverture allongée, sur un seul ou, préférentiellement, sur ses deux côtés.

Les zones en matériaux élastiques peuvent être formées d'au moins un élastomère thermoplastique d'origine naturelle ou synthétique. Le ou les élastomères mis en oeuvre peuvent être choisis préférentiellement dans le groupe constitué par des élastomères de type caoutchoucs naturels, caoutchouc synthétique, en particulier les caoutchoucs de mono-oléfines, tels que, par exemple, les polymères d'isobutylène/isoprène, éthylène-acétate de vinyle (EVA), éthylène-propylène (EPR), éthylène-propylène-diène (EPDM), éthylène-esters acryliques (EMA-EEA), les polymères fluorés, les caoutchouc de dioléfines, tels que, par exemple, les polybutadiène, les copolymères de butadiène-styrène (SBR), les caoutchoucs à base de produits de condensation tels que, par exemple, les caoutchoucs thermoplastiques polyesters et polyuréthanes, les silicones, les caoutchoucs styréniques, tels que styrène-butadiène-styrène (SBS) et styrène-isoprène-styrène (SIS) et autres, mis en oeuvre seuls ou en mélange.

Le dispositif de distribution (1) a préférentiellement la forme d'un disque tel que représenté sur les figures mais il peut avoir de multiples formes afin de s'adapter à divers types de conteneurs, et peut, par exemple, avoir une forme ovale ou carrée ou encore avoir une forme hémisphérique.

Des surfaces de guidage (6) des objets à distribuer (13) sont positionnées sur tout ou partie des arêtes des ouvertures (2, 3), du côté de la face antérieure, c'est-à-dire du côté du dispositif de distribution (1) orienté face au stock d'objets à distribuer (13). Ces surfaces de guidage (6) sont positionnées sur chaque arête, de part et d'autre des ouvertures allongées (2), et orientées du côté des objets à distribuer (13), ces objets étant stockés dans le conteneur (4).

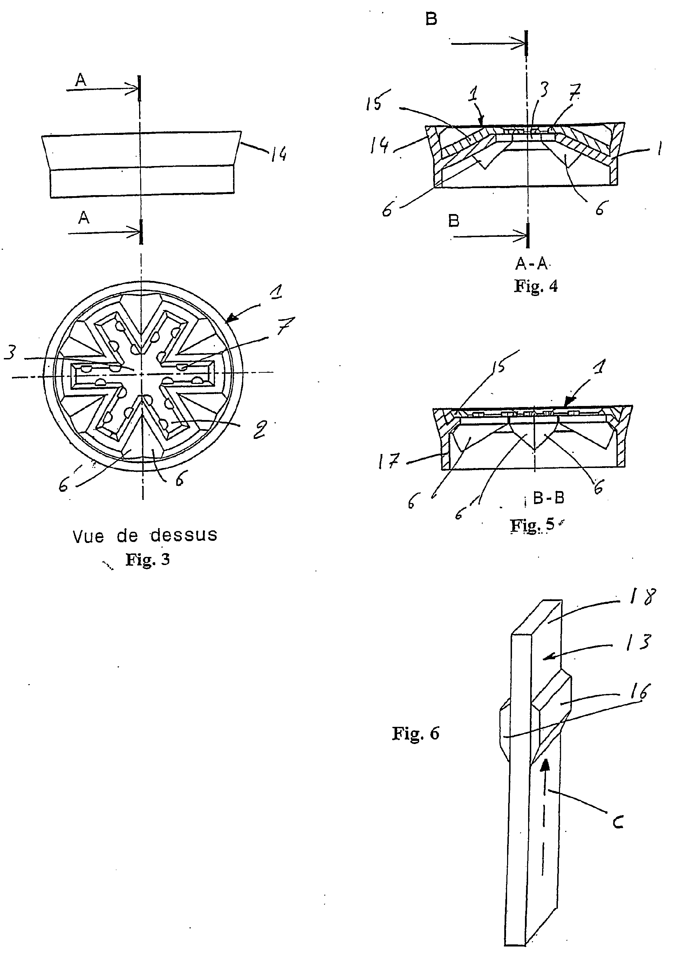

Les surfaces de guidage (6) sont des plans inclinés par rapport à l'ouverture (2) de manière à former une sorte de trémie permettant de collecter les objets à distribuer et dont la sortie est constituée par l'ouverture allongée (2). L'inclinaison des surfaces de guidage (6) par rapport au plan passant par les deux côtés de l'ouverture (2) est généralement comprise entre 20° et 60° et préférentiellement de 45°. Les surfaces de guidage (6, 6') de deux ouvertures allongées (2, 2') voisines et consécutives (représentées sur les figures 3 à 5) forment, en général, un dièdre ayant une arête vive (15) qui permet d'orienter les objets à distribuer vers l'une ou l'autre des ouvertures voisines (2, 2').

Les surfaces de guidage (6) positionnées de chaque côté des ouvertures allongées (2) sont préférentiellement planes mais peuvent être des surfaces à section curviligne en creux ou en relief, ou avoir toutes autres formes permettant la fonction de guidage des objets en direction desdites ouvertures allongées.

Afin de favoriser la fonction de guidage, les surfaces de guidage sont préférentiellement constituées de matériaux rigides qui permettent le glissement aisé des objets à distribuer.

Le dispositif de distribution (1) est préférentiellement réalisé en matériau polymère rigide moulé par injection de polymères et comporte les formes fonctionnelles importantes de l'invention que sont :
- les ouvertures allongées (2);
- l'ouverture principale de distribution (3);
- les surfaces de guidage (6) orientées du côté des objets à distribuer (13) et généralement placées de part et d'autre de chacune des ouvertures allongées ;
- si nécessaire, les éléments d'assemblage du dispositif de distribution (1) avec le conteneur (4).

Les matériaux polymères souhaitables pour réaliser le dispositif de distribution et plus particulièrement les surfaces de guidage sont généralement et non exclusivement choisis dans le groupe de matériaux composés de polymères thermoplastiques, comprenant en particulier les polyoléfines telles que les polyéthylènes, les polypropylènes, les copolymères d'éthylène/propylène et leurs mélanges, les polyamides (PA), les polystyrènes (PS), les copolymères d'acrylonitrile-butadiène-styrène (ABS), les copolymères de styrène-acrylonitrile (SAN), les polyméthacrylates de méthyl (PMMA), les polyéthylènetéréphtalates (PET), les polychlorures de vinyle, les polycarbonates.

Là ou les portions du dispositif de distribution réalisées en matériau(x) élastique(s) (15) sont généralement moulées sur la partie du dispositif de distribution en matériau rigide (17), la partie rigide (17) constituant généralement la structure principale du dispositif de distribution (1). Le matériau élastique est préférablement assemblé sous la forme d'une couche (15) dans laquelle sont formés les rebords élastiques disposés sur le pourtour des ouvertures allongées (2) et/ou de l'ouverture principale de distribution (3).

La figure 6 décrit un objet oblong, de type parallélépipédique à tendance plane (13) à distribuer par le dispositif de distribution (1).

Les objets oblongs à tendance plane à distribuer sont sensiblement identiques entre eux et ont la forme de lamelles parallélépipédiques, de tiges à section elliptique, à section polygonale, à section circulaire.

Il a été constaté que la qualité de la distribution unitaire des objets peut être améliorée en utilisant des lamelles parallélépipédiques disposant d'une légère excroissance (16) sur l'une au moins des deux faces planes de l'objet (13).

Il a également été constaté que cette excroissance doit être préférablement éloignée de l'extrémité de l'objet qui doit être insérée en premier dans l'ouverture principale de distribution (3), de manière à ce que l'objet (13) qui serait partiellement inséré dans une ouverture allongée (2) vienne en butée dans l'ouverture allongée (2) et ne puisse normalement sortir que par l'ouverture principale de distribution (3). L'excroissance (16) de l'objet (13) est généralement positionnée au tiers avant de l'objet à distribuer, du côté le plus proche du dispositif de distribution (1). Ce côté est indiqué par la flèche « C » désignant le sens de distribution de l'objet.

Afin d'améliorer la qualité de la distribution des objets, il est préférable d'orienter ces objets dans un seul sens (sens de la flèche « C ») de manière à favoriser un comportement relativement homogène de ces objets lors de l'opération de distribution. Pour cela, le stock d'objets à distribuer est généralement placé dans un conteneur (4) dont les dimensions intérieures permettent de conserver l'orientation relative des objets (13) par rapport au dispositif de distribution (1).

Le conteneur (4) a pour fonction principale de conserver le stock d'objets à distribuer à l'abri de perturbations externes qui peuvent être l'humidité, la poussière, les dégradations mécaniques tels que des chocs, ou autres.

Pour cela le conteneur (4) est généralement réalisé en matériaux polymères rigides, étanches à l'humidité. De tels matériaux peuvent être choisis dans le groupe constitué par les matériaux polymères thermoplastiques comprenant en particulier les polyoléfines telles que les polyéthylènes, les polypropylènes, les copolymères d'éthylène/propylène et leurs mélanges, les polyamides, les polystyrènes (PS), les copolymères d'acrylonitrile-butadiène-styrène (ABS), les copolymères de styrène-acrylonitrile (SAN), les polyméthacrylates de méthyl (PMMA), les polyéthylènetéréphtalates (PET), les polychlorures de vinyle, les polycarbonates.

Comme précédemment mentionné, et représenté sur la figure 1, le dispositif de distribution (1) est généralement assemblé à l'intérieur du conteneur (4) contenant les objets à distribuer (13), en formant un ensemble de stockage et de distribution unité par unité d'objets oblongs. Un tel mode d'assemblage est préféré car il permet de réaliser indépendamment le dispositif de distribution (1) et le conteneur (4) autorisant ainsi l'interchangeabilité du dispositif de distribution sur plusieurs types de conteneurs, et facilitant les opérations de chargement et rechargement du conteneur en objets à distribuer.

Plusieurs types d'assemblages entre le conteneur (4) et le dispositif de distribution (1) peuvent être utilisés. Par exemple, il est possible d'utiliser un assemblage par vissage, collage, clipsage ou par emmanchement du dispositif de distribution (1) à l'intérieur du conteneur (4). Dans le cas de l'assemblage par emmanchement, ledit conteneur est muni d'une butée pour bloquer en position le dispositif de distribution (1). Un emmanchement conique est généralement préféré car il permet à la fois la mise en position du dispositif par rapport au conteneur, mais aussi le blocage mécanique des pièces assemblées. Par exemple, le dispositif de distribution des figures 4 et 5 a une forme circulaire et possède une portion de surface externe conique (18) destinée à venir en appui sur une surface interne complémentaire et conique du conteneur (4). L'assemblage du dispositif de distribution (1) avec le conteneur (4) est réalisé de manière à positionner le dispositif de distribution à proximité de l'ouverture du conteneur (4) libérant ainsi un espace important pour le stockage des objets à distribuer (13).

Selon l'invention et pour assurer la fonction de protection contre l'humidité des objets à distribuer, il est également possible de munir le conteneur (4) d'un bouchon amovible (8) généralement fixé au conteneur par une charnière (9) dont l'axe de rotation est sensiblement perpendiculaire à l'axe principal du conteneur (4). Le bouchon (8) peut également se positionner sur le conteneur par tout autre moyen de fixation conventionnel comme des clips, une liaison par vissage du bouchon sur le conteneur, un assemblage par emboîtements.

Une autre protection particulière contre l'humidité peut également être réalisée par la présence d'élément(s) absorbant (s) ou dessicant(s) réalisés à partir de compositions dessicantes formées de mélanges de polymères et/ou copolymères thermoplastiques et/ou des élastomères pris seuls ou en mélange et de matériaux dessicants. Ces éléments dessicants peuvent se présenter sous la forme d'inserts, de couches de matériaux moulées sur la surface interne du conteneur (4) ou intégrées dans le matériau formant les parois du conteneur, et/ou insérés dans le bouchon (8) fermant le conteneur et/ou formant le bouchon.

Les éléments dessicants peuvent également former les matériaux constitutifs des parois du conteneur (4) et ou du bouchon (8).
Quant aux compositions dessicantes formées de mélanges de polymères et/ou copolymères thermoplastiques et de matériaux dessicants, les polymères et/ou copolymères mis en oeuvre sont choisis parmi ceux mis en oeuvre pour la réalisation du dispositif et du conteneur pris seuls ou en mélange, éventuellement associés à au moins un élastomère mis en oeuvre dans la réalisation des zones de contact des ouvertures allongées (2).

Le matériau dessicant est généralement choisi parmi les types de matériaux suivants.

Un premier type de matériaux dessicants comprend des composants chimiques qui peuvent se combiner avec de l'eau pour former des hydrates. De tels dessicants peuvent être :
- des sels anhydres qui ont tendance à capter l'eau et l'humidité en formant des sels hydratés,
- des oxydes anhydres réagissant avec de l'eau ou de la vapeur d'eau (humidité atmosphérique) pour former de nouveaux composés hydratés tels que par exemple l'oxyde de calcium, de magnésium, ou autres.

Un autre type de matériaux dessicants a pour capacité d'absorber l'humidité par action de capillarité liée à la morphologie du matériau. De tels matériaux sont principalement des silices, en particulier les gels de silice, des argiles telles que la Montmorillonite, les tamis moléculaires, de l'amidon, et certains matériaux synthétiques tels que des polybutadiènes, polysiloxanes.

Le bouchon (8) est préférentiellement muni d'une rainure d'obturation (11) venant se positionner sur une lèvre d'obturation (10) du conteneur (4), de manière à assurer une étanchéité de l'ensemble lorsque le conteneur (4) est bouché par le bouchon (8).

Le dispositif de distribution (1) précédemment décrit a pour fonction importante d'acheminer les objets (13) à distribuer vers l'ouverture principale de distribution (3). Cet acheminement se fait en plusieurs étapes :
- dans un premier temps l'utilisateur retourne ou secoue le dispositif de distribution de manière à ce que les objets à distribuer, mis en mouvement par inertie, viennent au contact mécanique du dispositif de distribution disposé au-dessous des objets à distribuer ;
- les objets ont généralement des mouvements relativement chaotiques et se trouvent dans de multiples positions. La présence de surfaces de guidage (6) inclinées disposées sur les arêtes d'une ouverture allongée, du côté du stock d'objets à distribuer, permet de positionner et d'aligner certains de ces objets par rapport à l'ouverture allongée (2) ;
- les objets (13) à distribuer ainsi positionnés et alignés le long des ouvertures allongées (2) ont tendance à glisser sous l'effet de leurs inerties. Le glissement se fait généralement le long de l'ouverture allongée (2) et en direction de l'ouverture principale de distribution (3) ;
- si aucun objet à distribuer n'est engagé dans l'ouverture principale de distribution (3), alors l'un des objets guidés le long de l'une des ouvertures allongées vient se positionner et s'engager dans l'ouverture principale de distribution (3);
- l'objet à distribuer ainsi positionné obture partiellement l'ouverture principale de distribution, évitant ainsi le passage d'un autre objet ;
- l'objet en cours de distribution reste généralement positionné et bloqué dans l'ouverture principale de distribution (3), ce blocage étant préférentiellement accentué par la présence de zones élastiques (7) disposées sur le pourtour de l'ouverture principale de distribution (3) et créant un frottement, freinant puis immobilisant progressivement l'objet en cours de distribution ;
- l'utilisateur peut alors aisément collecter l'objet partiellement distribué et accessible du côté extérieur du dispositif de distribution. En général, le blocage de l'objet dans l'ouverture principale de distribution est suffisant pour immobiliser l'objet dans cette ouverture et permettre à l'utilisateur de retourner ou positionner le dispositif de distribution de manière à faciliter la préhension de l'objet distribué.

Tous les détails de l'invention qui apparaissent au cours de la description numérotée des figures numéro 1 à 6 constituent une bonne illustration de son objet sans pour autant en réduire la portée.

## Revendications

1. Dispositif de distribution (1) unité par unité d'objets (13) de forme oblongue à section en particulier sensiblement polygonale, circulaire ou elliptique ou associées, comportant
une ouverture principale de distribution (3) permettant le passage des objets à distribuer (13) ; et
au moins une autre ouverture de forme allongée (2) dont l'un des côtés débouche dans l'ouverture principale de distribution (3), **caractérisé en ce que** l'ouverture allongée (2) a une largeur d'ouverture supérieure ou égale à l'épaisseur minimale de l'objet à distribuer ; et **en ce qu'**il comporte
des surfaces de guidage (6, 6') des objets à distribuer (13), constituées par des plans inclinés formant une trémie, lesdites surfaces de guidage étant disposées sur les côtés du dispositif orientés face au stock d'objet à distribuer de la ou des ouverture(s) allongée(s)(2), positionnées sur chaque arête, de part et d'autre des ouvertures allongées (2) et orientées face aux objets à distribuer (13), de manière à collecter et à guider lesdits objets à distribuer en direction de l'une au moins des ouvertures allongées (2).

2. Dispositif selon la revendication 1, **caractérisé en ce qu'**il possède plusieurs ouvertures allongées disposées radialement par rapport à l'axe de l'ouverture principale de distribution (3).

3. Dispositif (1) selon l'une quelconque des revendications précédentes, **caractérisé en ce que** la ou les ouvertures allongées (2) sont en forme de fentes.

4. Dispositif (1) selon l'une quelconque des revendications précédentes **caractérisé en ce que** la ou les ouverture(s) allongée(s) (2) en forme de fente, ont au moins un des côtés courbés, deux côtés courbés symétriquement, les côtés courbés formant un rétreint.

5. Dispositif (1) selon l'une quelconque des revendications précédentes, **caractérisé en ce que** la ou les ouvertures allongées (2) sont des fentes de largeurs substantiellement constantes.

6. Dispositif (1) selon l'une quelconque des revendications précédentes, **caractérisé en ce que** la ou les ouvertures allongées (2) sont des fentes dont la largeur est croissante en allant en direction de l'ouverture principale de distribution (3).

7. Dispositif (1) selon l'une quelconque des revendications précédentes **caractérisé en ce que** les ouvertures de forme allongée ont une longueur au moins égale à deux fois la largeur des objets à distribuer et préférentiellement égale à trois fois la largeur desdits objets à distribuer.

8. Dispositif (1) selon l'une quelconque des revendications précédentes, **caractérisé en ce que** l'ouverture principale de distribution (3) est une ouverture de forme circulaire dont le diamètre est proche de la largeur minimale des objets à distribuer (13).

9. Dispositif (1) selon l'une quelconque des revendications précédentes, **caractérisé en ce que** le rebord de l'une au moins des ouvertures comporte au moins une portion (7) en matériau élastique destinée à permettre le maintien par une légère tenue de l'objet distribué dans l'ouverture.

10. Dispositif (1) selon la revendication 9, **caractérisé en ce que** le matériau élastique est compris dans le groupe de matériaux constitué par des élastomères de type caoutchoucs naturels, caoutchouc synthétique, en particulier les caoutchoucs de mono-oléfines, dont les polymères d'isobutylène/isoprène, éthylène-acétate de vinyle (EVA), éthylène-propylène (EPR), éthylène-propylène-diène (EPDM), éthylène-esters acryliques (EMA-EEA), les polymères fluorés, en particulier les caoutchouc de dioléfines, dont les polybutadiènes, les copolymères de butadiène-styrène (SBR), en particulier les caoutchoucs à base de produits de condensation, dont les caoutchoucs thermoplastiques polyesters et polyuréthanes, les silicones, les caoutchoucs styréniques, en particulier styrène-butadiène-styrène (SBS) et styrène-isoprène-styrène (SIS), mis en oeuvre seuls ou en mélange.

11. Dispositif (1) selon l'une quelconque des revendications précédentes, **caractérisé en ce qu'**il a la forme d'un disque.

12. Dispositif (1) selon l'une quelconque des revendications précédentes, **caractérisé en ce que** les surfaces de guidage des objets à distribuer (13) sont constituées en matériau rigide permettant le glissement des objets à distribuer.

13. Dispositif (1) selon, l'une quelconque des revendications précédentes, **caractérisé en ce que** le matériau rigide constituant la surface de guidage est l'un au moins des matériaux du groupe de matériaux constitué par les polyéthylènes, les polypropylènes, les copolymères d'éthylène/propylène et leurs mélanges, les polyamides, les polystyrènes (PS), les copolymères, d'acrylonitrile-butadiène-styrène (ABS), les copolymères de styrène-acrylonitrile (SAN), les polyméthacrylates de méthyl .(PMMA), les polyéthylènetéréphtalates (PET), les polychlorures de vinyle, les polycarbonates.

14. Conteneur (4) contenant les objets à distribuer et possédant au moins une ouverture sur laquelle est monté le dispositif de distribution (1) de l'une quelconque des revendications 1 à 13.

15. Conteneur (4) de distribution d'objets selon la revendication précédente, **caractérisé en ce que** le dispositif de distribution (1) est assemblé à l'intérieur du conteneur (4).

16. Conteneur (4) de distribution d'objets selon la revendication précédente, **caractérisé en ce que** le dispositif de distribution (1) est assemblé avec le conteneur par un assemblage de type emmanchement conique, vissage, clipsage, collage.

17. Conteneur (4) de distribution selon l'une quelconque des revendications 14 à 16, **caractérisé en ce que** le conteneur est obturé de façon étanche par un bouchon amovible (8).

18. Conteneur (4) selon la revendication 17, **caractérisé en ce que** le bouchon (8) est articulé sur le conteneur par l'intermédiaire d'une charnière (9) dont l'axe est sensiblement perpendiculaire à l'axe du conteneur.

19. Conteneur (4) selon l'une quelconque des revendications 14 à 18, **caractérisé en ce qu'**il est formé de l'un au moins des matériaux polymères thermoplastiques appartenant au groupe de matériaux constitué par les polyéthylènes, les polypropylènes, les copolymères d'éthylène/propylène et leurs mélanges, les polyamides, les polystyrènes (PS), les copolymères d'acrylonitrile-butadiène-styrène (ABS), les copolymères de styrène-acrylonitrile (SAN), les polyméthacrylates de méthyl (PMMA), les polyéthylènetéréphtalates (PET), les polychlorures de vinyle, les polycarbonates.

20. Conteneur (4) selon l'une quelconque des revendications 14 à 19, **caractérisé en ce qu'**il comporte au moins un élément dessicant.

21. Conteneur (4) selon la revendication 20, **caractérisé en ce que** l'élément dessicant est choisi dans le groupe constitué par les inserts dessicants placés dans le conteneur, les couches dessicantes moulées sur la surface interne du conteneur et/ou du bouchon.

22. Conteneur (4) selon la revendication 20, **caractérisé en ce que** l'élément dessicant constitue les parois du conteneur et/ou du bouchon.

23. Conteneur (4) selon l'une quelconque des revendications 20 à 22, **caractérisé en ce que** l'élément dessicant est réalisé à partir de compositions dessicantes formées de mélanges de polymères et/ou copolymères thermoplastiques et de matériaux minéraux dessicants.

24. Conteneur (4) selon l'une quelconque des revendications 20 à 23, **caractérisé en ce que** les polymères et/ou copolymères thermoplastiques formant l'élément dessicant sont choisis parmi ceux formant ledit conteneur pris seuls ou en mélange, éventuellement associés à au moins un élastomère utilisés dans la réalisation des zones élastiques de contact des ouvertures allongées.

25. Conteneur (4) selon l'une quelconque des revendications 20 à 24, **caractérisé en ce que** les matériaux dessicants entrant dans la composition des éléments dessicants sont choisis dans le groupe constitué par les sels et oxydes métalliques anhydres susceptibles de réagir avec de la vapeur d'eau, et par les gels de silice, les tamis moléculaires, l'amidon, la Montmorillonite, les polybutadiènes et les polysiloxanes.

26. Conteneur (4) selon l'une quelconque des revendications 14 à 25 **caractérisé en ce qu'**il est assemblé avec le dispositif défini par l'une quelconque des revendications 1 à 13, en formant un ensemble de conditionnement et de distribution d'objets oblongs.

27. Utilisation du conteneur (4) selon les revendications 14 à 26 muni du dispositif de distribution selon les revendications 1 à 13 pour la distribution d'objets de forme oblongue tels que des languettes, des « strip » tests en forme de bandelettes ou lamelles rigides utilisées à des fins de diagnostic ou de contrôle dans le domaine médical.

## Claims

1. Device (1) for unit-by-unit dispensing of objects (13) with an oblong shape with a cross section that is in particular substantially polygonal, circular or elliptic or similar, comprising a main dispensing opening (3) allowing the passage of the objects to be dispensed (13); and at least one other opening with an elongated shape (2) one side of which opens into the main dispensing opening (3), **characterised in that** the elongated opening (2) has an opening width greater than or equal to the minimum thickness of the object to be dispensed, and **in that** it comprises guiding surfaces (6, 6') for the objects to be dispensed (13), said guiding surfaces having inclined planes forming a hopper, and said guiding surfaces being arranged on the sides of the device facing the stock of objects to be dispensed from the elongated opening or openings (2), positioned on each edge, on either side of the elongated openings (2) and facing the objects to be dispensed (13), so as to collect and guide said objects to be dispensed in the direction of at least one of the elongated openings (2).

2. Device according to claim 1, **characterised in that** it comprises several elongated openings arranged radially with respect to the axis of the principle dispensing opening (3).

3. Device (1) according to any one of the preceding claims, **characterised in that** the elongated opening(s) (2) are in the form of slots.

4. Device (1) according to any one of the preceding claims, **characterised in that** the elongated opening or openings (2) in the form of a slot have at least one curved edge, two sides curved symmetrically, the curved sides forming a constriction.

5. Device (1) according to any one of the preceding claims, **characterised in that** the elongated opening or openings (2) are slots with a substantially constant width.

6. Device (1) according to any one of the preceding claims, **characterised in that** the elongated opening or openings (2) are slots, whose width increases in the direction of the principle dispensing opening (3)

7. Device (1) according to any one of the preceding claims, **characterised in that** the openings with an elongated form have a length at least equal to twice the width of the objects to be dispensed and preferably equal to three times the width of said objects to be dispensed.

8. Device (1) according to any one of the preceding claims, **characterised in that** the principal dispensing opening (3) is an opening with a circular shape, whose diameter is close to the minimum width of the objects to be dispensed (13).

9. Device (1) according to any one of the preceding claims, **characterised in that** the edge of at least one of the openings comprises at least one portion (7) made of an elastic material designed to enable support with a slight holding of the object dispensed in the opening.

10. Device (1) according to claim 9, **characterised in that** the elastic material is one of a group of materials consisting of natural rubber elastomers, synthetic rubber, in particular monoolefine rubbers, including isobutylene/isoprene polymers, ethylene vinyl acetate (EVA), ethylene propylene rubber(EPR), ethylene propylene diene (EPDM), acrylic ethylene esters (EMA, EEA), fluorinated polymers, in particular diolefine rubbers, including polybutadienes, copolymers of styrene butadiene (SBR), in particular rubbers based on condensation products, including polyester thermoplastic rubbers and polyurethanes, silicones, styrenic rubbers, in particular styrene-butadiene styrene (SBS) and styrene isoprene styrene (SIS) used singly or as a mixture.

11. Device (1) according to any one of the preceding claims, **characterised in that** it has the shape of a disc.

12. Device (1) according to any one of the preceding claims, **characterised in that** the guiding surfaces of the objects to be dispensed (13) are made of a rigid material enabling the sliding of the objects to be dispensed.

13. Device (1) according to any one of the preceding claims, **characterised in that** the rigid material forming the guiding surface is at least one of the materials from a group of materials consisting of polyethylenes, polypropylenes, copolymers of ethylene/propylene and mixtures thereof, polyamides, polystyrenes (PS), copolymers of acrylonitrile-butadiene styrene (ABS), copolymers of styrene acrylonitrile (SAN), polymethylmethacrylates (PMMA), polyethyleneterephthalates (PET), polyvinyl chloride, polycarbonates.

14. Container (4) containing objects to be dispensed and having at least one opening on which the dispensing device (1) of any one of claims 1 to 13 is mounted.

15. Container (4) for dispensing objects according to the preceding claim, **characterised in that** the dispensing device (1) is assembled on the interior of the container (4).

16. Container (4) for dispensing objects according to the preceding claims, **characterised in that** the dispensing device (1) is connected to the container by means of a conical fitting, threaded joint, clipping or adhesive type of coupling.

17. Container (4) for dispensing according to any one of claims 14 to 16, **characterised in that** the container is closed off in a sealing manner by a removable cap (8).

18. Container (4) according to claim 17, **characterised in that** the plug (8) is articulated onto the container by means of a hinge (9) the axis of which is substantially perpendicular to the axis of the container.

19. Container (4) according to any one of claims 14 to 18, **characterised in that** it is made from at least one of the thermoplastic polymer materials belonging to a group of materials consisting of polyethylenes, polypropylenes, copolymers of ethylene/propylene and mixtures thereof, polyamides, polystyrenes (PS), copolymers of acrylonitrile-butadiene-styrene (ABS), copolymers of styrene acrylonitrile (SAN), polymethylmethacrylates (PMMA), polyethyleneterephthalates (PET), polyvinyl chlorides, polycarbonates.

20. Container (4) according to any one of claims 14 to 19, **characterised in that** it comprises at least one desiccant element.

21. Container (4) according to claim 20, **characterised in that** the desiccant element is selected from the group consisting of desiccant inserts placed in the container, the desiccant layers being moulded on the internal surface of the container and/or of the cap.

22. Container (4) according to claim 20, **characterised in that** the desiccant element forms the walls of the container and/or cap.

23. Container (4) according to any one of claims 20 to 22, **characterised in that** the desiccant element is made from desiccant compositions formed from mixtures of polymers and/or thermoplastic copolymers and desiccant mineral materials.

24. Container (4) according to any one of claims 20 to 23, **characterised in that** the thermoplastic polymers and/or copolymers forming the desiccant element are selected among those forming the said container taken singly or in a mixture, possibly associated with at least one elastomer used in the production of elastic contact zones of the elongated openings.

25. Container (4) according to any one of claims 20 to 24, **characterised in that** the desiccant materials entering into the composition of desiccant elements are selected from a group consisting of anhydrous salts and metal oxides capable of reacting with the water vapour, and by silica gels, molecular sieves, starch, montmorillonite, polybutadienes and polysiloxanes.

26. Container (4) according to any one of claims 14 to 25 **characterised in that** it is assembled with the device defined by any one of the claims 1 to 13, forming an assembly for packaging and dispensing oblong objects.

27. Use of the container (4) according to claims 14 to 26 equipped with the dispensing device according to claims 1 to 13 for dispensing objects with an oblong shape such as tabs, "strip" tests in the form of bandages or rigid strips used for diagnostic purposes or for control purposes in the medical field.

## Patentansprüche

1. Vorrichtung (1) zum Spenden einzelner länglicher Gegenstände (13) mit im Wesentlichen vieleckigen, kreisrunden oder elliptischen oder assoziierten bzw. ähnlichen Querschnitten, umfassend:
eine Spenderhauptöffnung (3) für den Durchgang der gespendeten Gegenstände (13); und wenigstens eine weitere Öffnung (2) von länglicher Form, die mit einer ihrer Seiten in der Spenderhauptöffnung (3) mündet,
**dadurch gekennzeichnet, dass** die Öffnungsbreite der länglichen Öffnung (2) größer als oder gleich groß wie die minimale Dicke des gespendeten Gegenstands ist; und **dadurch**, dass sie umfasst:
Flächen (6, 6') zur Führung der gespendeten Gegenstände (13), gebildet durch schräge Ebenen, die einen Trichter bilden - wobei die genannten Führungsflächen sich auf der dem Vorrat der von der (den) längliche(n) Öffnung(en) (2) gespendeten Gegenstände zugewandten Seite der Vorrichtung befinden -, und an jeder Kante beiderseits der länglichen Öffnungen (2) so positioniert und auf die gespendeten Gegenstände (13) ausgerichtet sind, dass sie die gespendeten Gegenstände sammeln und wenigstens einer der länglichen Öffnungen (2) zuführen.

2. Vorrichtung nach Anspruch 1, **dadurch gekennzeichnet, dass** sie mehrere längliche Öffnungen besitzt, die in Bezug auf die Achse der Spenderhauptöffnung (3) radial angeordnet sind.

3. Vorrichtung (1) nach einem der vorangehenden Ansprüche, **dadurch gekennzeichnet, dass** die längliche Öffnung oder länglichen Öffnungen (2) schlitzförmig sind.

4. Vorrichtung (1) nach einem der vorangehenden Ansprüche, **dadurch gekennzeichnet, dass** die schlitzförmige längliche Öffnung oder länglichen Öffnungen (2) wenigstens eine gekrümmte Seite, zwei symmetrisch gekrümmte Seiten aufweisen, wobei die gekrümmten Seiten eine Verengung bilden.

5. Vorrichtung (1) nach einem der vorangehenden Ansprüche, **dadurch gekennzeichnet, dass** die längliche Öffnung oder länglichen Öffnungen (2) Schlitze von im Wesentlichen konstanter Breite sind.

6. Vorrichtung (1) nach einem der vorangehenden Ansprüche, **dadurch gekennzeichnet, dass** die längliche Öffnung oder länglichen Öffnungen (2) Schlitze sind, deren Breite in Richtung Spenderhauptöffnung (3) zunimmt.

7. Vorrichtung (1) nach einem der vorangehenden Ansprüche, **dadurch gekennzeichnet, dass** die Länge der länglichen Öffnungen wenigstens doppelt so groß wie die Breite der gespendeten Gegenstände und vorzugsweise dreimal so groß wie die Breite der gespendeten Gegenstände ist.

8. Vorrichtung (1) nach einem der vorangehenden Ansprüche, **dadurch gekennzeichnet, dass** die Spenderhauptöffnung (3) eine kreisförmige Öffnung ist, deren Durchmesser ungefähr der minimalen Breite der gespendeten Gegenstände (13) entspricht.

9. Vorrichtung (1) nach einem der vorangehenden Ansprüche, **dadurch gekennzeichnet, dass** der Rand von wenigstens einer der Öffnungen wenigstens einen Ansatz (7) aus elastischem Material umfasst, der dazu dient, die gespendeten Gegenständen in der Öffnung leicht festzuhalten.

10. Vorrichtung (1) nach Anspruch 9, **dadurch gekennzeichnet, dass** das elastische Material in der Materialgruppe enthalten ist, die gebildet wird durch Elastomere des Typs natürliche Kautschuke, synthetische Kautschuke, insbesondere die Monoolefinkautschuke, davon die Isobutylen/Isopren-Polymere, Ethylen-Vinylacetat (EVA), Ethylen-Propylen (EPR), Ethylen-Propylen-Dien (EPDM), Ethylen-Acrylsäureestern (EMA-EEA), die Fluorpolymere, insbesondere die Diolefinkautschuke, davon die Polybutadiene, die Butadien-Styrol-Copolymere (SBR), insbesondere die Kautschuke auf der Basis von Kondensationsprodukten, davon die thermoplastischen Polyester- und Polyurethankautschuke, die Silicone, die Styrolkautschuke, insbesondere Styrol-Butadien-Styrol (SBS) und Styrol-Isopren-Styrol (SIS), einzeln oder als Mischung verwendet.

11. Vorrichtung (1) nach einem der vorangehenden Ansprüche, **dadurch gekennzeichnet, dass** sie scheibenförmig ist.

12. Vorrichtung (1) nach einem der vorangehenden Ansprüche, **dadurch gekennzeichnet, dass** die Flächen zur Führung der gespendeten Gegenstände (13) aus einem steifen Material sind, das ein Gleiten der gespendeten Gegenstände ermöglicht.

13. Vorrichtung (1) nach einem der vorangehenden Ansprüche, **dadurch gekennzeichnet, dass** das die Führungsflächen bildende Material wenigstens eines der Materialien aus der Materialgruppe ist, die gebildet wird durch die Polyethylene, die Polypropylene, die Ethylen/Propylen-Copolymere und ihre Mischungen, die Polyamide, die Polystyrole (PS), die Acrylnitril-Butadien-Styrol-Copolymere (ABS), die Styrol-Acrylnitril-Copolymerisate (SAN), die Polymethylmethacrylate (PMMA), die Polyethylenterephthalate (PET), die Polyvinylchloride, die Polycarbonate.

14. Behälter (4), der die zu spendenden Gegenstände enthält und wenigstens eine Öffnung besitzt, auf welche die Spendervorrichtung (1) nach einem der Ansprüche 1 bis 13 montiert wird.

15. Behälter (4) zum Spenden von Gegenständen nach dem vorangehenden Anspruch, **dadurch gekennzeichnet, dass** die Spendervorrichtung (1) in den Behälter (4) montiert wird.

16. Behälter (4) zum Spenden von Gegenständen nach dem vorangehenden Anspruch, **dadurch gekennzeichnet, dass** die Spendervorrichtung (1) mit dem Behälter zusammengebaut wird durch eine Montage des Typs konischer Einpresssitz, Clip, Schraubung, Klebung.

17. Behälter (4) nach einem der Ansprüche 14 bis 16, **dadurch gekennzeichnet, dass** der Behälter dicht verschlossen wird durch einen abnehmbaren Deckel (8).

18. Behälter (4) nach Anspruch 17, **dadurch gekennzeichnet, dass** der Deckel (8) mit dem Behälter gelenkig verbunden ist durch ein Scharnier (9), dessen Achse im Wesentlichen senkrecht ist zur Achse des Behälters.

19. Behälter (4) nach einem der Ansprüche 14 bis 18, **dadurch gekennzeichnet, dass** er aus wenigstens einem der thermoplastischen polymeren Materialien ist, die zu der Materialgruppe gehören, die gebildet wird durch die Polyethylene, die Polypropylene, die Ethylen/Propylen-Copolymere und ihre Mischungen, die Polyamide, die Polystyrole (PS), die Acrylnitril-Butadien-Styrol-Copolymere (ABS), die Styrol-Acrylnitril-Copolymerisate (SAN), die Polymethylmethacrylate (PMMA), die Polyethylenterephthalate (PET), die Polyvinylchloride, die Polycarbonate.

20. Behälter (4) nach einem der Ansprüche 14 bis 19, **dadurch gekennzeichnet, dass** er wenigstens ein Trocknungselement umfasst.

21. Behälter (4) nach Anspruch 20, **dadurch gekennzeichnet, dass** das Trocknungselement aus der Gruppe gewählt wird, die gebildet wird durch in den Behälter gelegte Trocknungseinsätze, auf die Innenoberfläche des Behälters und/oder Deckels gegossene Trocknungsschichten.

22. Behälter (4) nach Anspruch 20, **dadurch gekennzeichnet, dass** das Trocknungselement die Wände des Behälters und/oder des Deckels bildet.

23. Behälter (4) nach einem der Ansprüche 20 bis 22, **dadurch gekennzeichnet, dass** das Trocknungselement aus Trocknungszusammensetzungen realisiert wird, die durch Mischungen von thermoplastischen Polymeren und/oder Copolymeren und mineralischen Trocknungsmaterialien gebildet werden.

24. Behälter (4) nach einem der Ansprüche 20 bis 23, **dadurch gekennzeichnet, dass** die das Trocknungselement bildenden thermoplastischen Polymere und/oder Copolymere ausgewählt werden unter denen, die den Behälter bilden, einzeln oder als Mischung verwendet, eventuell in Verbindung mit wenigstens einem Elastomer, benutzt zur Realisierung der elastischen Kontaktzonen der länglichen Öffnungen.

25. Behälter (4) nach einem der Ansprüche 20 bis 24, **dadurch gekennzeichnet, dass** die Trocknungsmaterialien, die für die Zusammensetzung der Trocknungselemente verwendet werden, aus der Gruppe gewählt werden, die gebildet wird durch die mit Wasserdampf reagierenden wasserfreien Salze und Metalloxide und die Kieselgels, die Molekularsiebe, das Amidon, das Montmorillonit, die Polybutadiene und die Polysiloxane.

26. Behälter (4) nach einem der Ansprüche 14 bis 25, **dadurch gekennzeichnet, dass** er zusammengebaut wird mit der durch einen der Ansprüche 1 bis 13 definierten Vorrichtung, um eine Einheit zum Verpacken und Spenden von länglichen Gegenständen zu bilden.

27. Verwendung des Behälters (4) nach den Ansprüchen 14 bis 26, ausgestatte mit der Spendervorrichtung nach den Ansprüchen 1 bis 13 zum Spenden von länglichen Gegenständen wie Streifen (languettes), streifenförmigen "Teststrips" oder steifen Lamellen bzw. Blättchen zu Diagnose- oder Kontrollzwecken im medizinischen Bereich.
